# FASCICULE DE BREVET EUROPEEN

(11) **EP 0 670 157 B1**
(45) Date de publication et mention de la délivrance du brevet: **01.10.1997**
(21) Numéro de dépôt: 95400134.3
(22) Date de dépôt: 23.01.1995
(51) Int. Cl.: A61K 7/48, A61K 7/06, A61K 31/375

(54) **Emulsion contenant de l'acide ascorbique stabilisé**
Emulsion, die stabilisierte Ascorbinsäure enthält
Emulsion containing stabilised ascorbic acid

(30) Priorité: 04.02.1994 FR 9401282
(43) Date de publication de la demande: 06.09.1995
(73) Titulaire: L'OREAL, 75008 Paris (FR)
(72) Inventeur: Candau, Didier, F-91570 Bievres (FR); Collin, Nathalie, F-92330 Sceaux (FR)
(74) Mandataire: Lhoste, Catherine

(56) Documents cités:
- WO-A-94/09756
- US-A- 5 051 252

## Description

L'invention se rapporte à une émulsion eau dans huile (E/H) contenant de l'acide ascorbique stabilisé, utilisable en particulier dans les domaines cosmétique, dermatologique et/ou vétérinaire.

L'invention se rapporte aussi à une utilisation de cette émulsion pour le traitement cosmétique de la peau ainsi que pour la préparation d'une crème ou pommade destinée au traitement dermatologique de la peau et/ou au traitement vétérinaire.

L'invention se rapporte encore à un procédé de traitement cosmétique qui consiste à appliquer sur la peau ladite émulsion.

L'émulsion de l'invention peut être appliquée, par voie topique, sur le visage, y compris autour des yeux, sur le corps ainsi que sur le cuir chevelu des êtres humains.

On cherche depuis longtemps à stabiliser l'acide ascorbique, ou vitamine C, dans des présentations galéniques appropriées, du fait de ses propriétés bénéfiques.

En effet, l'acide ascorbique possède de nombreuses fonctions biologiques comme la stimulation de la synthèse du collagène, le renfort des tissus cutanés contre les agressions extérieures (rayonnements UV, pollution), la dépigmentation, l'activité anti-radicaux libres, la compensation de la déficience en vitamine E. Certaines de ces propriétés bénéfiques ont été rapportées notamment par ENGLAND ET SEIFTER dans l'article "The biochemical functions of ascorbic acid" paru dans Ann. Rev. Nutri.,1986 : 6, pp 365-406.

Cependant, en raison de sa structure chimique (d'alpha-cétolactone) l'acide ascorbique est très sensible à l'influence des paramètres de l'environnement comme la lumière, l'oxygène, l'eau (par son pH et par la présence de trace de métaux). Il s'ensuit une dégradation inéluctable au cours du temps de l'acide ascorbique en solution.

Dans l'état de la technique ce problème a été diversement traité.

Pour diminuer ou retarder la dégradation de l'acide ascorbique en solution, les auteurs du document US-A-5140043 ont préconisé de le stabiliser en l'introduisant dans des solutions hydroalcooliques, formées d'au moins 80 % d'eau et ayant un pH inférieur à 3,5.

En raison de la forte acidité de ces solutions, leur utilisation dans le domaine cosmétique et/ou pharmaceutique est difficilement envisageable.

En effet, une application répétée de ces solutions peut perturber l'équilibre de la peau et en particulier irriter, voire brûler la peau.

On connaît, par ailleurs, l'article de B.R. Hajratwala intitulé "Stability of ascorbic acid", paru dans la Revue Sciences Pharmaceutiques, le 15 Mars 1985.

Dans cet article il est enseigné notamment de stabiliser l'acide ascorbique en solution aqueuse acide par ajout d'un agent tensioactif qui est un ester de sorbitanne oxyéthyléné.

En particulier, l'auteur y a rapporté qu'à pH 3,4 et à 25 °C, l'ajout de cet agent diminuait la vitesse d'oxydation, donc de dégradation de l'acide ascorbique en solution.

En outre, ce document enseigne l'emploi d'agent chélatant tel que l'éthylène diamine tétraacétique (EDTA) et le conditionnement sous azote, en l'absence de lumière, pour améliorer la stabilité de l'acide ascorbique en solution aqueuse.

Une telle solution aqueuse acide, appliquée sur la peau, présente les mêmes inconvénients que ceux décrits ci-dessus pour des solutions hydroalcooliques acides. De plus, la stabilisation obtenue est encore insuffisante.

D'autres modes de stabilisation de l'acide ascorbique ont été envisagés notamment par enrobage (technique décrite dans le document FR-A-1600826) ou par granulation de l'acide ascorbique (technique illustrée dans le document JP-A-53-127819, pour l'agro-alimentaire).

Mais ces techniques sont d'une part coûteuses et peuvent d'autre part altérer l'acide ascorbique, par exemple lors d'un chauffage, et/ou conduire à des compositions peu cosmétiques, comme c'est le cas des granulés.

On connaît, par ailleurs, du document FR-A-1489249 l'emploi de sels métalliques d'acide ascorbique phosphorylé, notamment l'ascorbylphosphate de magnésium, dans des compositions cosmétiques.

Ce dernier composé a une activité proche de celle de l'acide ascorbique dont il est issu mais il présente certains inconvénients qui rendent son utilisation sur la peau peu probable. En particulier, l'ascorbylphosphate de magnésium n'étant stable qu'en pH basique (pH 8 à pH 9), il doit être incorporé dans une composition basique qui peut être irritante pour la peau (dont le pH a une valeur d'environ 5,5).

En conséquence, l'ensemble des propositions qui ont été faites jusqu'ici n'a pas permis de résoudre les problèmes techniques liés à l'instabilité de l'acide ascorbique en solution, dans une forme galénique appropriée pour les domaines cosmétiques et/ou dermatologiques et à un coût compatible avec les exigences industrielles.

Il subsiste donc le besoin d'une composition, utilisable dans les domaines cosmétique, dermatologique et/ou vétérinaire, contenant de l'acide ascorbique stabilisé, à l'état libre, c'est à dire sans groupement additionnel notamment stabilisant, et qui ne provoque aucune irritation de la peau, après application.

La demanderesse a maintenant trouvé une composition du type émulsion eau dans huile permettant d'atteindre ces objectifs.

Cette émulsion comprend une phase aqueuse, dispersée dans une phase huileuse à l'aide d'un agent émulsionnant, caractérisée en ce que la phase aqueuse contient de l'acide ascorbique stabilisé et présente un pH acide, de valeur au plus égale à 3,5, et en ce que l'agent émulsionnant est un diméthiconecopolyol et/ou un alkyldiméthiconecopolyol.

Dans ce qui suit l'agent émulsionnant utilisé dans l'invention sera défini selon la nomenclature CTFA.

L'intérêt d'utiliser une telle émulsion E/H réside notamment, dans le fait d'avoir la phase aqueuse acide, contenant l'acide ascorbique, en faible quantité sur la peau, à l'état de fines gouttelettes dispersées dans l'huile, ce qui ne provoque pas d'irritation ni de brûlure de la peau. L'émulsion de l'invention est par conséquent bien supportée par les utilisateurs à l'inverse des solutions.

Les concentrations en acide ascorbique dans l'émulsion de l'invention sont celles classiquement utilisées dans le domaine cosmétique et par exemple de 0,1 % à 10 %, et de préférence de 0,5 % à 5 % en poids par rapport au poids total de l'émulsion.

Par ailleurs, le fait que l'émulsion de l'invention incorpore de l'acide ascorbique à l'état libre, protoné, procure des traitements plus efficaces par rapport aux préparations de l'art antérieur qui comportent des "dérivés" d'acide ascorbique hydrolysables au contact de la peau.

L'émulsion de l'invention peut se présenter sous forme de lait ou de crème utilisable en particulier dans les domaines cosmétique, dermatologique et/ou vétérinaire. Elle possède une texture légère et s'étale bien. Elle donne de plus, à l'application, une sensation de fraîcheur et procure un éclat du teint instantané. Elle permet, en particulier, un lissage des traits et des imperfections de la peau.

D'autre part, l'émulsion conforme à l'invention présente l'avantage de pouvoir inclure tout type d'actif hydrophile ou lipophile, insensible aux acides, autre que l'acide ascorbique.

Par ailleurs, l'émulsion de l'invention contenant comme émulsionnant un diméthiconecopolyol et/ou alkyldiméthiconecopolyol présente une meilleure stabilité, à température ambiante (20°C), que celle présentée par des émulsions contenant d'autres types d'émulsionnants.

Cet émulsionnant comprend de préférence des groupements polyéthers totalement oxyéthylénés, mais on peut utiliser des émulsionnants partiellement oxyéthylénés.

De manière avantageuse, le pourcentage en poids de polyéther par rapport au poids total de l'agent émulsionnant est choisi de 1 % à 50 %, de préférence de 15 % à 35 % dans le cas des diméthiconecopolyols et de 1 % à 5 %, de préférence de 2 % à 3 % dans le cas des alkyldiméthiconecopolyols.

Comme diméthiconecopolyol on peut citer le produit vendu sous la dénomination Q2-3225C par la Société DOW CORNING. Toutefois, on utilise de préférence le produit vendu sous la dénomination SF-1228 par la Société GENERAL ELECTRIC.

Comme alkyldiméthiconecopolyol on peut utiliser le lauryldiméthiconecopolyol vendu, par exemple, sous la dénomination Q2-5200 par la Société DOW CORNING. Toutefois, on utilise de préférence le cétyldiméthiconecopolyol vendu, par exemple, sous la dénomination ABIL EM 90 par la Société GOLDSCHMIDT.

Les agents émulsionnants utilisables dans l'émulsion de l'invention peuvent être associés en outre à au moins un coémulsionnant tel que, notamment, le tétraisostéarate de polyglycérol ou le trioléate de polyglycérol.

Ces agents émulsionnants sont en pratique présents à raison de 1 % à 10 %, et de préférence de 2,5 % à 3,5 % en poids par rapport au poids total de l'émulsion lorsqu'ils sont employés seuls. Lorsque la teneur en agent émulsionnant est choisie inférieure à 2,5 % en poids par rapport au poids total de l'émulsion, il est préférable d'ajouter un coémulsionnant. Quand il est présent le coémulsionnant est utilisé à raison de 1 fois à 10 fois la quantité de l'agent émulsionnant. De manière avantageuse, les concentrations respectives d'agents émulsionnants et coémulsionnants sont choisies de 0,5 % à 2,2 % et de 3 % à 7 % en poids par rapport au poids total de l'émulsion.

Pour des températures supérieures à 20°C et/ou pour des longues périodes de stockage, de plusieurs mois, on préfère utiliser comme agent émulsionnant un alkyldiméthiconecopolyol totalement oxyéthyléné et plus spécialement, le cétyldiméthiconecopolyol.

Pour que le pH de la phase aqueuse de l'émulsion selon l'invention reste acide pendant de longues périodes, on associe avantageusement à l'acide ascorbique un acide additionnel stable. Cet acide peut être tout acide permettant d'obtenir l'effet tampon acide recherché.

De préférence cet acide est un α-hydroxy-acide choisi parmi l'acide lactique, l'acide glycolique et l'acide citrique.

En effet, ce type particulier d'acide présente l'avantage supplémentaire, dans le cas où il est appliqué sur la peau, d'éliminer les cellules mortes de l'épiderme par son action kératolytique et de favoriser la pénétration d'autres actifs notamment, celle de l'acide ascorbique.

Comme autres acides on peut utiliser notamment les β-hydroxy-acides ou l'acide acétique.

Par ailleurs la quantité d'acide, que ce soit l'acide ascorbique seul ou en mélange avec un acide additionnel, doit être suffisante pour obtenir une valeur de pH acide et en particulier de 1,5 à 3,5, et de préférence de 1,8 à 2,7.

Pour atteindre les valeurs préférées de pH (1,8 à 2,7) l'acide ascorbique, s'il est employé seul, doit être présent à une concentration supérieure à 4 % en poids par rapport au poids total de l'émulsion.

Selon l'invention, la phase aqueuse de l'émulsion peut représenter de 60 % à 80 %, et, de préférence de 65 % à 75 % en poids par rapport au poids total de l'émulsion. En dessous de 60 %, il peut y avoir relargage d'huile et l'émulsion n'est alors plus homogène. Au-dessus de 80 %, l'émulsion obtenue est plus difficile à étaler sur la peau en raison de sa viscosité.

La phase aqueuse de l'émulsion de l'invention peut, en outre, renfermer un électrolyte, comme par exemple le chlorure de sodium ou de potassium, de manière à stabiliser encore plus l'émulsion.

La teneur en électrolyte, lorsqu'il est présent, est en général de 0,5 % à 2 % en poids par rapport au poids total de l'émulsion.

De façon avantageuse, et afin d'éviter notamment la présence dans la phase aqueuse de métaux lourds pouvant catalyser la dégradation de l'acide ascorbique, la phase aqueuse est formée d'eau permutée ou désionisée.

Pour augmenter encore la stabilité de l'acide ascorbique au cours du temps, l'émulsion de l'invention peut comprendre un agent séquestrant les métaux tel qu'un dérivé d'acide phosphonique.

Les dérivés d'acide phosphonique utilisables dans l'invention sont notamment choisis parmi l'acide éthylènediamine tétra(méthylène phosphonique), l'acide hexaméthylènediamine tétra(méthylène phosphonique), l'acide diéthylènetriamine penta(méthylènephosphonique), et leurs sels et notamment leur sels de sodium comme le sel pentasodique de l'acide éthylènediamine tétra(méthylène phosphonique).

De manière avantageuse, on utilise l'acide éthylènediamine tétra(méthylène phosphonique), notamment vendu par la Société MONSANTO sous la dénomination Dequest 2041. On peut aussi utiliser avantageusement le sel pentasodique de ce dernier qui est vendu sous la dénomination Dequest 2046 par la Société MONSANTO. Comme autre agent séquestrant utilisable dans l'émulsion de l'invention on peut citer l'acide diéthylène triamine pentaacétique, vendu par exemple par la Société SIGMA.

Le choix de l'agent séquestrant les métaux n'est pas indifférent. Il est établi, et la demanderesse a pu le constater dans l'émulsion selon l'invention, qu'en présence de fer, l'éthylène diamine tétraacétique (EDTA) a un effet prooxydant et donc de déstabilisation de l'acide ascorbique, ce qui est indésirable.

Lorsqu'il est présent, l'agent séquestrant a une concentration allant en général de 0,05 % à 0,2 % en poids par rapport au poids total de l'émulsion.

La phase huileuse de l'émulsion de l'invention peut renfermer toute sorte d'huiles et de corps gras bien connus de l'homme du métier, comme les huiles minérales, organiques, végétales ou synthétiques, siliconées ou non et en particulier, les huiles de silicone volatiles, une fraction liquide du beurre de karité (triglycérides d'acide palmitique, stéarique, oléique, linoléique), l'huile d'amande, l'huile d'abricot, le mélange d'éthyl-2 hexanoate de cétylstéaryle et de myristate d'isopropyle, le perhydrosqualène de synthèse.

De préférence, la phase huileuse de l'émulsion de l'invention comprend une huile siliconée volatile généralement à raison de 3 % à 15 % en poids par rapport au poids total de l'émulsion comme, par exemple, une silicone cyclique telle que la cyclopentadiméthylsiloxane. Cette dernière est notamment vendue par la Société DOW CORNING sous la dénomination D₅. On peut aussi utiliser tout autre huile siliconée volatile et notamment la cyclohexadiméthylsiloxane vendue sous la dénomination D₆ par la Société DOW CORNING.

La phase huileuse peut comprendre, en outre, un agent gélifiant, habituellement à raison de 1 % à 15 %, de préférence 3 % à 7 % du poids total de l'émulsion. Lorsqu'il est présent, cet agent gélifiant peut être, en particulier, un dérivé d'une argile de type hectorite, comme celui vendu sous la dénomination de Simagel Si 345 par la Société STEARINERIES DUBOIS.

L'émulsion de l'invention peut comprendre enfin tout additif, insensible aux acides, compatible avec l'application visée.

Cet additif, lorsqu'il est présent, a notamment une concentration communément admise dans les domaines considérés notamment en fonction de son degré de solubilité. De manière générale, cette concentration peut être choisie de 0,01 % à 30 % en poids par rapport au poids total de l'émulsion.

Comme additif on peut citer des actifs hydrophiles ou lipophiles, des émollients liposolubles, des conservateurs, des parfums, des charges, des matières colorantes, à condition bien entendu que l'additif ne déstabilise pas l'acide ascorbique dans l'émulsion.

Lorsqu'il est présent, l'émollient liposoluble peut être en particulier une gomme de silicone comme le mélange de cyclopentadiméthylsiloxane et de diméthiconol pour son apport de douceur.

Les actifs utilisables en particulier dans l'invention comprennent notamment les agents hydratants tels que le glycérol, le pyrrolidone carboxylate de sodium, les NMF (facteurs normaux d'hydratation), et l'acide hyaluronique.

D'autres actifs peuvent être utilisés, et notamment les filtres UV, les protéines ou hydrolysats de protéines, les extraits végétaux, les huiles essentielles.

De façon avantageuse, la composition de l'invention comprend, en poids :
- de 0,5 % à 5 % d'acide ascorbique,
- de 2,5 % à 3,5 % de cétyldiméthiconecopolyol,
- de 3 % à 15 % d'huile siliconée volatile,
- de 0,01 % à 0,1 % de sel pentasodique de l'acide éthylène tétra(méthylène phosphonique), et
- de 0,5 % à 2 % de chlorure de sodium.

Comme déja mentionné ci-dessus, l'acide ascorbique est instable en présence de lumière et d'oxygéne. C'est pourquoi il est préférable que l'émulsion selon l'invention soit conditionnée de sorte à ne pas être en contact avec l'oxygène et à être à l'abri de la lumière .

Aussi l'émulsion de l'invention est de préférence préparée sous atmosphère inerte (azote ou gaz rare tel que l'argon ), exempte de tout oxygène et sous lumière inactinique, telle que celle d'une lampe à vapeur de sodium.

De manière avantageuse, l'émulsion de l'invention est conditionnée en présence d'un absorbeur d'oxygène. Ce dernier est de préférence séparé de l'émulsion par une membrane poreuse aux gaz et imperméable aux liquides.

Comme exemple d'absorbeur d'oxygène utilisable dans l'émulsion selon l'invention on peut citer, l'absorbeur d'oxygène Atco vendu par la Société STANDA INDUSTRIES.

En tant que membrane poreuse aux gaz et imperméable aux liquides utilisable dans la présente invention, on peut citer celle décrite dans le document FR-A-2671055.

De manière encore plus préférée, l'émulsion de l'invention est conditionnée dans un conteneur surmonté d'un dispositif de distribution sans reprise d'air comme, par exemple, celui décrit dans le document FR-A-2666308.

L'invention a aussi pour objet une composition cosmétique, dermatologique et/ou vétérinaire consistant en une émulsion telle que définie précédemment.

L'invention a encore pour objet une utilisation de l'émulsion ci-dessus pour le traitement cosmétique de la peau et en particulier pour lisser les ridules de la peau, la tonifier, la régénérer, pour éclaircir le teint, éliminer les taches pigmentaires de la peau, pour lutter contre les méfaits des rayonnements UV, et/ou pour renforcer de manière gènèrale les tissus cutanés contre les agressions de l'environnement (pollution).

L'invention a également pour objet l'utilisation de l'émulsion ci-dessus pour la fabrication d'une crème destinée à un traitement dermatologique et/ou vétérinaire.

L'invention a enfin pour objet un procédé de traitement cosmétique qui consiste à appliquer sur la peau, y compris autour des yeux, une émulsion conforme à l'invention.

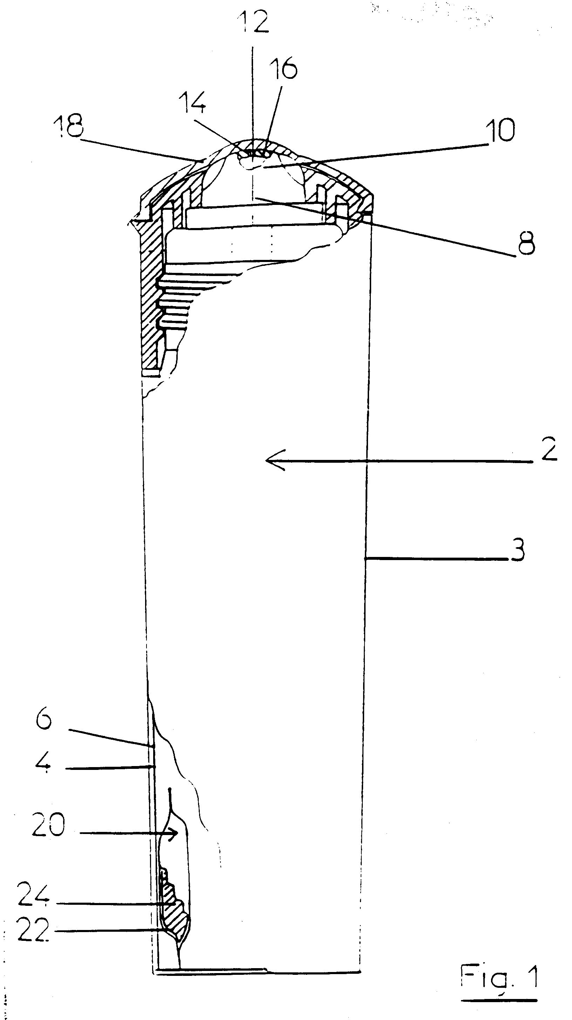

D'autres avantages et caractéristiques de l'invention apparaîtront mieux à la lecture de la description qui suit, donnée à titre illustratif, en référence à l'unique figure annexée, la figure 1, et aux différents exemples. La figure 1 représente un exemple de conditionnement compatible avec l'émulsion selon l'invention.

Le conditionnement (2) comporte un conteneur (3) dont les parois (4) sont constituées par tout matériau imperméable aux gaz pouvant être fait, par exemple, d'un matériau thermoformé ou de tout autre matériau approprié à condition que la surface interne desdites parois, en contact avec l'émulsion (6), soit non métallique et que ce matériau ne laisse pas pénétrer la lumière.

Le conteneur (3) est surmonté d'un dispositif de distribution d'air (8), constitué d'un dôme convexe vers l'extérieur (10) et d'au moins une fente (12) prévue au sommet du dôme.

Les parois (14, 16) de la fente (12) sont propres à venir en contact l'une de l'autre pour réaliser une fermeture étanche, au repos. L'ensemble du dispositif (8) est surmonté d'un capot hermétique (18) assurant une double protection, de l'émulsion de l'invention vis à vis de l'environnement.

Une poche (20) indépendante du conditionnement, dont les parois (22) sont formées d'une membrane poreuse aux gaz et imperméable aux liquides, contient un absorbeur d'oxygène (24). La membrane (22) peut être formée d'un matériau choisi parmi le polyéthylène, le propylène et polyéthylène/téréphtalate.

Au repos, les parois (14, 16) du dôme sont en contact étroit, assurant ainsi l'étanchéité de la fente (12). En exerçant une pression sur le conditionnement (2), on transmet à l'émulsion de l'invention une pression qui aura tendance à écarter les parois (14, 16) permettant ainsi à l'émulsion de sortir du conditionnement. La pression étant relachée, les parois (14, 16) retrouvent leur position de repos et ferment de manière occlusive la fente (12).

On donne ci-après des exemples de compositions, pour le soin, selon l'invention. Les compositions qui suivent ont été obtenues selon le mode opératoire suivant.

On réalise successivement les étapes 1 à 3 suivantes dans une boîte à gants et sous atmosphère d'azote en opérant en permanence par dégazage ainsi qu'en présence de lumière inactinique et à température ambiante (20°C) :
1) On dissout les constituants de la phase aqueuse (A) sous agitation modérée puis on ajoute l'acide ascorbique qui est préalablement solubilisé par agitation au barreau aimanté juste avant de faire l'émulsion.
2) Par ailleurs, on mélange les constituants de la phase huileuse (B).
3) On verse alors la phase (A) dans la phase (B) et on agite violemment par cisaillement à l'aide d'un mélangeur de type Moritz pendant 1 min à 10 min, puis on effectue une agitation douce, à la pale, pour obtenir une émulsion selon l'invention qui se présente, en particulier, sous forme de crème.

Dans ces émulsions E/H stockées pendant au moins 2 mois à température ambiante, on ne note aucune dégradradation de l'acide ascorbique. Ainsi ce dernier a bien été stabilisé.

Pour des périodes de stockage plus longues, l'acide ascorbique dans ces émulsions s'est également montré particulièrement stable et son pourcentage de dégradation s'est avéré nettement inférieur à ceux de l'art antérieur.

Par ailleurs, on constate une dégradation de seulement 10 % à 20 % de l'acide ascorbique après deux mois de conservation à une température aussi élevée que 45°C.

### . Exemple 1 : Crème pour le visage

| | | |
|---|---|---|
| - Cétyldiméthiconecopolyol | | 3 |
| - Cyclopentadiméthylsiloxane | | 12 |
| - Mélange d'éthyl - 2 hexanoate de cétylstéaryle et de myristate d'isopropyle (90/10) | | 3 |
| - Triglycérides d'acide palmitique, stéarique, oléique, linoléique (5/25/60/10) | | 2 |
| - Hectorite modifiée par chlorure de distéaryldiméthylammonium, dispersion dans cyclopentadiméthylsiloxane et éthanol (15/80/5) | | 5 |
| - Sel pentasodique de l'acide éthylène tétra(méthylène phosphonique) dans l'eau à 33 % | | 0,1 |
| - Chlorure de sodium | | 0,7 |
| - Acide lactique | | 4 |
| - Acide ascorbique | | 1 |
| - Conservateurs | { Diazolidinylurée | 0,2 |
| | { Butyl paraben / Acide sorbique | 0,4 |
| - Parfum | | 0,3 |
| - Eau permutée qsp | | 100 |

Cette crème pour le soin du visage a une texture épaisse. Elle est nourrissante, douce à l'étalement. Elle procure un éclat du teint immédiat et permet un lissage des imperfections.

### . Exemple 2 : Crème translucide pour le corps

| | | |
|---|---|---|
| - Diméthiconecopolyol/Cyclopentadiméthylsiloxane (10/90) | | 25 |
| - Hectorite modifiée par chlorure de distéaryldiméthylammonium, dispersion dans cyclopentadiméthylsiloxane et éthanol (15/80/5) | | 5 |
| - Sel pentasodique de l'acide éthylène tétra(méthylène phosphonique) dans l'eau à 33 % | | 0,1 |
| - Chlorure de sodium | | 0,7 |
| - Acide glycolique | | 4 |
| - Acide ascorbique | | 1 |
| - Conservateurs | { Diazolidinylurée | 0,2 |
| | { Butyl paraben / Acide sorbique | 0,4 |
| - Parfum | | 0,3 |
| - Eau permutée qsp | | 100 |

Cette crème translucide pour le corps est très légère à l'étalement et pénètre instantanément dans la peau. Elle est tonifiante, régénérante, et nourrit la peau du corps.

### . Exemple 3 : Crème pour peaux grasses

| | | |
|---|---|---|
| - Cétyldiméthiconecopolyol | | 1 |
| - Tetraisostéarate de triglycéryle | | 5 |
| - Cyclopentadiméthylsiloxane | | 9 |
| - Cyclohexadiméthylsiloxane | | 4 |
| - Huile d'abricot | | 3 |
| - Mélange de cyclopentadiméthylsiloxane et de diméthiconol (90/10) | | 4 |
| - Glycérine | | 5 |
| - Acide glycolique | | 4 |
| - Acide ascorbique | | 1 |
| - Chlorure de sodium | | 0,7 |
| - Conservateurs | { Diazolidinylurée | 0,2 |
| | { Butyl paraben / Acide sorbique | 0,4 |
| - Parfum | | 0,3 |
| - Eau permutée qsp | | 100 |

Cette crème pour peaux grasses est de texture fluide. Elle est glissante, légère à l'étalement et réalise un lissage des traits. Elle procure un éclat du teint.

### . Exemple 4 : Crème pour peaux normales

| | | |
|---|---|---|
| - Cétyldiméthiconecopolyol | | 2 |
| - Trioléate de triglycéryle | | 5 |
| - Cyclopentadiméthylsiloxane | | 8 |
| - Cyclohexadiméthylsiloxane | | 4 |
| - Mélange de cyclopentadiméthylsiloxane | | |
| et de diméthiconol (90/10) | | 4 |
| - Huile d'abricot | | 3 |
| - Glycérine | | 3 |
| - Acide ascorbique | | 5 |
| - Chlorure de sodium | | 0,5 |
| - Conservateurs | { Diazolidinylurée | 0,2 |
| | { Butyl paraben / Acide sorbique | 0,4 |
| - Parfum | | 0,3 |
| - Eau permutée qsp | | 100 |

Cette crème de soin pour peaux normales est très confortable et filmogène après application. Elle procure un éclat du teint tout en permettant un lissage des imperfections de la peau.

## Revendications

1. Emulsion comprenant une phase aqueuse dispersée dans une phase huileuse à l'aide d'un agent émulsionnant, caractérisée en ce que la phase aqueuse contient de l'acide ascorbique stabilisé et présente un pH acide, de valeur au plus égale à 3,5, et en ce que l'agent émulsionnant est un diméthiconecopolyol et/ou un alkyldiméthiconecopolyol.

2. Emulsion selon la revendication 1, caractérisée en ce que l'acide ascorbique est présent à une teneur choisie de 0,5 % à 5 % en poids par rapport au poids total de l'émulsion.

3. Emulsion selon la revendication 1 ou 2, caractérisée en ce qu'elle comprend un acide additionnel autre que l'acide ascorbique.

4. Emulsion selon l'une quelconque des revendications 1 à 3, caractérisée en ce que la valeur du pH de la phase aqueuse est choisie de 1,5 à 3,5.

5. Emulsion selon l'une quelconque des revendications précédentes, caractérisée en ce que la valeur du pH de la phase aqueuse est choisie de 1,8 à 2,7.

6. Emulsion selon l'une quelconque des revendications 3 à 5, caractérisée en ce que le second acide est un α-hydroxy-acide.

7. Emulsion selon la revendication précédente, caractérisée en ce que l'α-hydroxy-acide est l'acide lactique.

8. Emulsion selon l'une quelconque des revendications précédentes, caractérisée en ce que la phase aqueuse représente de 60 % à 80 % en poids par rapport au poids total de l'émulsion.

9. Emulsion selon l'une quelconque des revendications précédentes, caractérisée en ce que la phase aqueuse représente de 65 % à 75 % en poids par rapport au poids total de l'émulsion.

10. Emulsion selon l'une quelconque des revendications précédentes, caractérisée en ce que l'agent émulsionnant est présent à raison de 1 % à 10 % en poids par rapport au poids total de l'émulsion.

11. Emulsion selon l'une quelconque des revendications précédentes, caractérisée en ce que l'agent émulsionnant comporte des groupements polyéthers totalement oxyéthylénés.

12. Emulsion selon l'une quelconque des revendications 1 à 11, caractérisée en ce que l'agent émulsionnant est un alkyldiméthiconecopolyol dont le pourcentage en poids des groupements polyéthers par rapport au poids total d'alkyldiméthiconecopolyol est de 2,5 %.

13. Emulsion selon l'une quelconque des revendications 1 à 12, caractérisée en ce que l'agent émulsionnant est un alkyldiméthiconecopolyol dont le groupement alkyle possède un nombre d'atomes égal à 16.

14. Emulsion selon l'une quelconque des revendications 1 à 11, caractérisée en ce que l'agent émulsionnant est un diméthiconecopolyol dont le pourcentage en poids de groupements polyéthers par rapport au poids total de diméthiconecopolyol est de 25 %.

15. Emulsion selon l'une quelconque des revendications précédentes, caractérisée en ce qu'elle contient, en outre, un coémulsionnant utilisé à raison de 1 fois à 10 fois la quantité de l'agent émulsionnant.

16. Emulsion selon l'une quelconque des revendications précédentes, caractérisée en qu'elle comprend, en outre, un agent séquestrant les métaux.

17. Emulsion selon la revendication précédente, caractérisée en ce que l'agent séquestrant est le sel pentasodique de l'acide éthylènediamine tétra(méthylène phosphonique).

18. Emulsion selon l'une quelconque des revendications 1 à 17, caractérisée en ce qu'elle est conditionnée de sorte à ne pas être au contact d'oxygène ni d'éléments métalliques et à être à l'abri de la lumière.

19. Emulsion selon l'une quelconque des revendications précédentes, caractérisée en ce qu'elle est conditionnée en présence d'un absorbeur d'oxygène séparé de ladite émulsion par une membrane poreuse aux gaz et imperméable aux liquides.

20. Emulsion selon l'une quelconque des revendications précédentes, caractérisée en ce qu'elle comprend, en poids:
- de 0,5 % à 5 % d'acide ascorbique,
- de 2,5 % à 3,5 % de cétyldiméthiconecopolyol,
- de 3 % à 15 % d'huile siliconée volatile,
- de 0,01 % à 0,1 % de sel pentasodique de l'acide éthylène tétra(méthylène phosphonique), et
- de 0,5 % à 2 % de chlorure de sodium.

21. Emulsion selon l'une quelconque des revendications précédentes, caractérisée en ce l'agent émulsionnant est présent à raison de 2,5 % à 3,5 % en poids par rapport au poids total de l'émulsion, en l'absence de coémulsionnant.

22. Emulsion selon l'une quelconque des revendications 1 à 20, caractérisée en ce que l'agent émulsionnant a une teneur inférieure à 2,5 %, en présence d'un coémulsionnant.

23. Composition cosmétique, dermatologique et/ou vétérinaire, caractérisée en ce qu'elle consiste en une émulsion selon l'une quelconque des revendications précédentes.

24. Utilisation d'une émulsion selon l'une quelconque des revendications 1 à 22 pour un traitement cosmétique de la peau en vue de la tonifier, de la régénérer, de lisser les ridules de la peau, d'éclaircir le teint, d'éliminer les taches pigmentaires de la peau, et/ou pour lutter contre les méfaits des rayonnements UV, et/ou pour renforcer les tissus cutanés contre les agressions de l'environnement.

25. Utilisation d'une émulsion selon l'une quelconque des revendications 1 à 22 pour la fabrication d'une crème destinée à un traitement dermatologique et/ou vétérinaire.

26. Procédé de traitement cosmétique caractérisé en ce qu'il consiste à appliquer sur la peau, y compris autour des yeux, une émulsion selon l'une quelconque des revendications 1 à 22.

## Claims

1. Emulsion comprising an aqueous phase dispersed in an oily phase using an emulsifying agent, characterized in that the aqueous phase contains stabilized ascorbic acid and has an acidic pH, which is at most equal to 3.5, and in that the emulsifying agent is a dimethiconecopolyol and/or an alkyldimethiconecopolyol.

2. Emulsion according to Claim 1, characterized in that the ascorbic acid is present in a content chosen from 0.5 % to 5 % by weight relative to the total weight of the emulsion.

3. Emulsion according to Claim 1 or 2, characterized in that it comprises an additional acid other than ascorbic acid.

4. Emulsion according to any one of Claims 1 to 3, characterized in that the pH value of the aqueous phase is chosen from 1.5 to 3.5.

5. Emulsion according to any one of the preceding claims, characterized in that the pH value of the aqueous phase is chosen from 1.8 to 2.7.

6. Emulsion according to any one of Claims 3 to 5, characterized in that the second acid is an α-hydroxy acid.

7. Emulsion according to the preceding claim, characterized in that the α-hydroxy acid is lactic acid.

8. Emulsion according to any one of the preceding claims, characterized in that the aqueous phase represents from 60 % to 80 % by weight relative to the total weight of the emulsion.

9. Emulsion according to any one of the preceding claims, characterized in that the aqueous phase represents from 65 % to 75 % by weight relative to the total weight of the emulsion.

10. Emulsion according to any one of the preceding claims, characterized in that the emulsifying agent is present in an amount of 1 % to 10 % by weight relative to the total weight of the emulsion.

11. Emulsion according to any one of the preceding claims, characterized in that the emulsifying agent contains totally oxyethylenated polyether groups.

12. Emulsion according to any one of Claims 1 to 11, characterized in that the emulsifying agent is an alkyl-dimethiconecopolyol, the percentage by weight of the polyether groups of which relative to the total weight of alkyldimethiconecopolyol is 2.5 %.

13. Emulsion according to any one of Claims 1 to 12, characterized in that the emulsifying agent is an alkyldimethiconecopolyol, the alkyl group of which has a number of atoms equal to 16.

14. Emulsion according to any one of Claims 1 to 11, characterized in that the emulsifying agent is a dimethiconecopolyol, the percentage by weight of polyether groups of which relative to the total weight of dimethiconecopolyol is 25 %.

15. Emulsion according to any one of the preceding claims, characterized in that it additionally contains a co-emulsifying agent used in an amount which is 1 to 10 times the amount of the emulsifying agent.

16. Emulsion according to any one of the preceding claims, characterized in that it additionally comprises a metal-sequestering agent.

17. Emulsion according to the preceding claim, characterized in that the sequestering agent is the pentasodium salt of ethylenediaminetetra(methylenephosphonic) acid.

18. Emulsion according to any one of Claims 1 to 17, characterized in that it is packaged so as not to be in contact with oxygen or with metal elements and so as to be protected from the light.

19. Emulsion according to any one of the preceding claims, characterized in that it is packaged in the presence of an oxygen trap which is separated from the said emulsion by a gas-porous and liquid-impermeable membrane.

20. Emulsion according to any one of the preceding claims, characterized in that it comprises, by weight:
- from 0.5 % to 5 % of ascorbic acid,
- from 2.5 % to 3.5 % of cetyldimethiconecopolyol,
- from 3 % to 15 % of volatile silicone-containing oil,
- from 0.01 % to 0.1 % of the pentasodium salt of ethylenetetra(methylenephosphonic) acid, and
- from 0.5 % to 2 % of sodium chloride.

21. Emulsion according to any one of the preceding claims, characterized in that the emulsifying agent is present in an amount of 2.5 % to 3.5 % by weight relative to the total weight of the emulsion, in the absence of co-emulsifying agent.

22. Emulsion according to any one of Claims 1 to 20, characterized in that the emulsifying agent is at a content below 2.5 %, in the presence of a co-emulsifying agent.

23. Cosmetic, dermatological and/or veterinary composition, characterized in that it consists of an emulsion according to any one of the preceding claims.

24. Use of an emulsion according to any one of Claims 1 to 22 for a cosmetic treatment of the skin in order to tone and regenerate it, to smooth out the fine lines in the skin, to lighten the complexion, to remove blemishes from the skin, and/or in order to combat the harmful effects of UV radiation, and/or in order to strengthen skin tissues against environmental attacks.

25. Use of an emulsion according to any one of Claims 1 to 22 for the manufacture of a cream intended for a dermatological and/or veterinary treatment.

26. Cosmetic treatment process, characterized in that it consists of application to the skin, including application around the eyes, of an emulsion according to any one of Claims 1 to 22.

## Patentansprüche

1. Emulsion, die eine durch einen Emulgator in einer Ölphase dispergierte wässerige Phase enthält,
dadurch gekennzeichnet, daß
die wässerige Phase stabilisierte Ascorbinsäure enthält und einen sauren pH-Wert von höchstens 3,5 aufweist und dadurch, daß der Emulgator ein Dimeticoncopolyol und/oder Alkyldimeticoncopolyol ist.

2. Emulsion nach Anspruch 1, dadurch gekennzeichnet, daß die Ascorbinsäure in einem Mengenanteil von 0,5 bis 5 Gew.-%, bezogen auf das Gesamtgewicht der Emulsion, vorliegt.

3. Emulsion nach Anspruch 1 oder 2, dadurch gekennzeichnet, daß sie eine von Ascorbinsäure verschiedene weitere Säure enthält.

4. Emulsion nach einem der Ansprüche 1 bis 3, dadurch gekennzeichnet, daß der pH-Wert der wässerigen Phase im Bereich von 1,5 bis 3,5 ausgewählt ist.

5. Emulsion nach einem der vorhergehenden Ansprüche, dadurch gekennzeichnet, daß der pH-Wert der wässerigen Phase im Bereich von 1,8 bis 2,7 ausgewählt ist.

6. Emulsion nach einem der Ansprüche 3 bis 5, dadurch gekennzeichnet, daß die zweite Säure eine α-Hydroxysäure ist.

7. Emulsion nach dem vorhergehenden Anspruch, dadurch gekennzeichnet, daß die α-Hydroxysäure die Milchsäure ist.

8. Emulsion nach einem der vorhergehenden Ansprüche, dadurch gekennzeichnet, daß die wässerige Phase 60 bis 80 Gew.-%, bezogen auf das Gesamtgewicht der Emulsion, ausmacht.

9. Emulsion nach einem der vorhergehenden Ansprüche, dadurch gekennzeichnet, daß die wässerige Phase 65 bis 75 Gew.-%, bezogen auf das Gesamtgewicht der Emulsion, ausmacht.

10. Emulsion nach einem der vorhergehenden Ansprüche, dadurch gekennzeichnet, daß der Emulgator in einem Mengenanteil von 1 bis 10 Gew.-%, bezogen auf das Gesamtgewicht der Emulsion, vorliegt.

11. Emulsion nach einem der vorhergehenden Ansprüche, dadurch gekennzeichnet, daß der Emulgator vollständig ethoxylierte Polyethergruppen enthält.

12. Emulsion nach einem der Ansprüche 1 bis 11, dadurch gekennzeichnet, daß der Emulgator ein Alkyldimeticoncopolyol ist, dessen Prozentsatz an Polyethergruppen, bezogen auf das Gesamtgewicht des Alkydimeticoncopolyols, 2,5 Gew.-% beträgt.

13. Emulsion nach einem der Ansprüche 1 bis 12, dadurch gekennzeichnet, daß der Emulgator ein Alkyldimeticoncopolyol ist, dessen Alkylgruppe 16 Atome aufweist.

14. Emulsion nach einem der Ansprüche 1 bis 11, dadurch gekennzeichnet, daß der Emulgator ein Dimeticoncopolyol ist, dessen Prozentsatz an Polyethergruppen, bezogen auf das Gesamtgewicht des Dimeticoncopolyols, 25 Gew.% beträgt.

15. Emulsion nach einem der vorhergehenden Ansprüche, dadurch gekennzeichnet, daß sie ferner einen Coemulgator enthält, der in einem Mengenanteil verwendet wird, der 1- bis 10-fach über der Menge des Emulgators liegt.

16. Emulsion nach einem der vorhergehenden Ansprüche, dadurch gekennzeichnet, daß sie ferner ein Maskierungsmittel für Metalle enthält.

17. Emulsion nach dem vorhergehenden Anspruch, dadurch gekennzeichnet, daß das Maskierungsmittel das Pentanatriumsalz von Ethylendiamintetramethylenphosphonsäure ist.

18. Emulsion nach einem der Ansprüche 1 bis 17, dadurch gekennzeichnet, daß sie so abgepackt ist, daß sie weder mit Sauerstoff noch mit metallischen Elementen in Kontakt steht und unter Lichtausschluß vorliegt.

19. Emulsion nach einem der vorhergehenden Ansprüche, dadurch gekennzeichnet, daß sie in Gegenwart eines Sauerstoffabsorbers abgepackt ist, der von der Emulsion über eine Membran getrennt ist, die gasdurchlässig und undurchlässig für Flüssigkeiten ist.

20. Emulsion nach einem der vorhergehenden Ansprüche, dadurch gekennzeichnet, daß sie enthält:
- 0,5 bis 5 Gew.-% Ascorbinsäure,
- 2,5 bis 3,5 Gew.-% Cetyldimeticoncopolyol,
- 3 bis 15 Gew.-% flüchtiges Siliconöl,
- 0,01 bis 0,1 Gew.-% Pentanatriumsalz von Ethylentetramethylenphosphonsäure, und
- 0,5 bis 2 Gew.-% Natriumchlorid.

21. Emulsion nach einem der vorhergehenden Ansprüche, dadurch gekennzeichnet, daß der Emulgator bei Abwesenheit eines Coemulgators in einem Mengenanteil von 2,5 bis 3,5 Gew.-%, bezogen auf das Gesamtgewicht der Emulsion, vorliegt.

22. Emulsion nach einem der Ansprüche 1 bis 20, dadurch gekennzeichnet, daß der Emulgator in Gegenwart eines Coemulgators in einem Mengenanteil unter 2,5 % vorliegt.

23. Kosmetische, dermatologische und/oder veterinärmedizinische Zusammensetzung, dadurch gekennzeichnet, daß sie aus einer Emulsion nach einem der vorhergehenden Ansprüche besteht.

24. Verwendung einer Emulsion nach einem der Ansprüche 1 bis 22 zur kosmetischen Behandlung der Haut, um die Haut zu beleben, zu regenerieren, die Falten der Haut zu glätten, den Teint aufzuhellen, Pigmentflecken der Haut zu beseitigen und/oder die schädlichen Wirkungen der UV-Strahlung zu bekämpfen und/oder das Hautgewebe gegen schädliche Umwelteinflüsse zu stärken.

25. Verwendung einer Emulsion nach einem der Ansprüche 1 bis 22 zur Herstellung einer Creme, die zur dermatologischen und/oder veterinärmedizinischen Behandlung bestimmt ist.

26. Verfahren zur kosmetischen Behandlung, dadurch gekennzeichnet, daß es darin besteht, eine Emulsion nach einem der Ansprüche 1 bis 22 auf die Haut einschließlich der Hautpartien um die Augen aufzutragen.
